# EUROPEAN PATENT SPECIFICATION

(11) **EP 2 271 642 B1**
(45) Date of publication and mention of the grant of the patent: **05.03.2014**
(21) Application number: 09731060.1
(22) Date of filing: 10.04.2009
(51) Int. Cl.: C07D 403/14, C07D 403/06, C07D 213/06, A61K 31/4418, A61K 31/4436, A61K 31/443, A61K 31/444, A61K 31/4439

(54) **PYRIDYL INHIBITORS OF HEDGEHOG SIGNALLING**
PYRIDYLHEMMER DES HEDGEHOG-SIGNALWEGES
INHIBITEURS PYRIDILES DE SIGNALISATION HEDGEHOG

(30) Priority: 11.04.2008 US 44451 P
(43) Date of publication of application: 12.01.2011
(73) Proprietor: Genentech, Inc., South San Francisco, CA 94080 (US); Curis, Inc., Lexington, MA 02421 (US)
(72) Inventor: GUNZNER, Janet, L., Piedmont CA 94611 (US); SUTHERLIN, Daniel, P., Burlingame CA 94010 (US); STANLEY, Mark, S., Pacifica CA 94044 (US); BAO, Liang, Daly City CA 94015 (US); CASTANEDO, Georgette, Redwood City CA 94061 (US); LALONDE, Rebecca, L., Berkeley CA 94704 (US); WANG, Shumei, Foster City CA 94404 (US); REYNOLDS, Mark, E., Millbrae CA 94030 (US); SAVAGE, Scott, J., Santa Clara CA 95054 (US); MALESKY, Kimberly, San Francisco CA 94133 (US); DINA, Michael, S., Daly City CA 94014 (US)
(74) Representative: Kilger, Ute
(86) International application number: PCT/US2009/040165
(87) International publication number: WO 2009/126863

(56) References cited:
- WO-A1-03/068747
- WO-A2-03/037274
- WO-A2-2004/058176
- WO-A2-2006/028958
- WO-A2-2006/028958

## Description

### PRIORITY CLAIM

This application claims priority to provisional United States application 61/044451 filed on 11 April 2008.

### FIELD OF THE INVENTION

The present invention relates to the field of organic compounds useful for methods of therapy and/or prophylaxis in a mammal, in particular to a certain pyridyl compound that inhibits the hedgehog signaling pathway and that is useful in methods of treatment of hyperproliferative diseases and angiogenesis mediated diseases.

### BACKGROUND OF THE INVENTION

Hedgehog (Hh) protein was first identified in Drosophila melanogaster as a segment-polarity gene involved in embryo patterning (Nusslein-Volhard et al., Roux. Arch. Dev. Biol. 193: 267-282 (1984)). Three orthologs of Drosophila hedgehog (Sonic, Desert and Indian) were later identified to occur in all vertebrates including fish, birds and mammals. Desert hedgehog (DHh) is expressed principally in the testes, both in mouse embryonic development and in the adult rodent and human; Indian hedgehog (IHh) is involved in bone development during embryogenesis and in bone formation in the adult; and, Sonic hedgehog (SHh) is expressed at high levels in the notochord and floor plate of developing vertebrate embryos. In vitro explant assays as well as ectopic expression of SHh in transgenic animals have shown that SHh plays a key role in neuronal tube patterning (Echelard et al., supra.; Ericson et al., Cell 81: 747-56 (1995); Marti et al., Nature 375: 322-5 (1995); Krauss et al., Cell 75, 1432-44 (1993); Riddle et al., Cell 75: 1401-16 (1993); Roelink et al, Cell 81:445-55 (1995); Hynes et al., Neuron 19: 15-26 (1997)). Hh also plays a role in the development of limbs (Krauss et al., Cell 75: 1431-44 (1993); Laufer et al., Cell 79, 993-1003 (1994)), somites (Fan and Tessier-Lavigne, Cell 79, 1175-86 (1994); Johnson et al., Cell 79: 1165-73 (1994)), lungs (Bellusci et al., Develop. 124: 53-63 (1997) and skin (Oro et al., Science 276: 817-21 (1997)). Likewise, IHh and DHh are involved in bone, gut and germinal cell development (Apelqvist et al., Curr. Biol. 7: 801-4 (1997); Bellusci et al., Dev. Suppl. 124: 53-63 (1997); Bitgood et al., Curr. Biol. 6: 298-304 (1996); Roberts et al., Development 121: 3163-74 (1995)).

Human SHh is synthesized as a 45 kDa precursor protein that upon autocatalytic cleavage yields a 20 kDa N-terminal fragment that is responsible for normal hedgehog signaling activity; and a 25 kDa C-terminal fragment that is responsible for auto-processing activity in which the N-terminal fragment is conjugated to a cholesterol moiety (Lee, J.J., et al. (1994) Science 266, 1528- 1536; Bumcrot, D.A., et al. (1995), Mol. Cell Biol. 15, 2294-2303; Porter, J.A., et al. (1995) Nature 374, 363- 366). The N-terminal fragment consists of amino acid residues 24-197 of the full-length precursor sequence which remains membrane-associated through the cholesterol at its C-terminus (Porter, J.A., et al. (1996) Science 274, 255- 258; Porter, J.A., et al. (1995) Cell 86, 21-34). Cholesterol conjugation is responsible for the tissue localization of the hedgehog signal.

At the cell surface, the Hh signal is thought to be relayed by the 12 transmembrane domain protein Patched (Ptc) (Hooper and Scott, Cell 59: 751-65 (1989); Nakano et al., Nature 341: 508-13 (1989)) and the G-protein-coupled-like receptor Smoothened (Smo) (Alcedo et al., Cell 86: 221-232 (1996); van den Heuvel and Ingham, Nature 382: 547-551 (1996)). Both genetic and biochemical evidence support a receptor model where Ptc and Smo are part of a multicomponent receptor complex (Chen and Struhl, Cell 87: 553-63 (1996); Marigo et al., Nature 384: 176-9 (1996); Stone et al., Nature 384: 129-34 (1996)). Upon binding of Hh to Ptc, the normal inhibitory effect of Ptc on Smo is relieved, allowing Smo to transduce the Hh signal across the plasma membrane. However, the exact mechanism by which Ptc controls Smo activity still has yet to be clarified.

The signaling cascade initiated by Smo results in activation of Gli transcription factors that translocate into the nucleus where they control transcription of target genes. Gli has been shown to influence transcription of Hh pathway inhibitors such as Ptc and Hip1 in a negative feedback loop indicating that tight control the Hh pathway activity is required for proper cellular differentiation and organ formation. Uncontrolled activation of Hh signaling pathway is associated with malignancies in particular those of the brain, skin and muscle as well as angiogenesis. An explanation for this is that Hh pathway has been shown to regulate cell proliferation in adults by activation of genes involved in cell cycle progression such as cyclin D which is involved in G1-S transition. Also, SHh blocks cell-cycle arrest mediated by p21, an inhibitor of cyclin dependent kinases. Hh signaling is further implicated in cancer by inducing components in the EGFR pathway (EGF, Her2) involved in proliferation as well as components in the PDGF (PDGFα) and VEGF pathways involved in angiogenesis. Loss of function mutations in the Ptc gene have been identified in patients with the basal cell nevus syndrome (BCNS), a hereditary disease characterized by multiple basal cell carcinomas (BCCs). Dysfunctional Ptc gene mutations have also been associated with a large percentage of sporadic basal cell carcinoma tumors (Chidambaram et al., Cancer Research 56: 4599-601 (1996); Gailani et al., Nature Genet. 14: 78-81 (1996); Hahn et al., Cell 85: 841-51 (1996); Johnson et al., Science 272: 1668-71 (1996); Unden et al., Cancer Res. 56: 4562-5; Wicking et al., Am. J. Hum. Genet. 60: 21-6 (1997)). Loss of Ptc function is thought to cause an uncontrolled Smo signaling in basal cell carcinoma. Similarly, activating Smo mutations have been identified in sporadic BCC tumors (Xie et al., Nature 391: 90-2 (1998)), emphasizing the role of Smo as the signaling subunit in the receptor complex for SHh.

Various inhibitors of hedgehog signaling have been investigated such as Cyclopamine, a natural alkaloid that has been shown to arrest cell cycle at G0-G1 and to induce apoptosis in SCLC. Cyclopamine is believed to inhibit Smo by binding to its heptahelical bundle. Forskolin has been shown to inhibit the Hh pathway downstream from Smo by activating protein kinase A (PKA) which maintains Gli transcription factors inactive.

Particularly, WO 2006/028958 A2 disclosed pyridyl inhibitors of hedgehog signaling useful in methods of treatment of cancer. However, the person skilled in the art is aware that depending on the selection of specific residues and on the selection of positions of their attachment, properties of the resultant compound can be modified unpredictably, e.g. its corresponding activity can be increased significantly to be useful in methods of treating respective diseases.

Despite advances with the above stated and other compounds, there remains a need for potent inhibitors of the hedgehog signaling pathway.

### SUMMARY OF THE INVENTION

In one aspect of the present invention there is provided a novel hedgehog inhibitor having the formula (I)

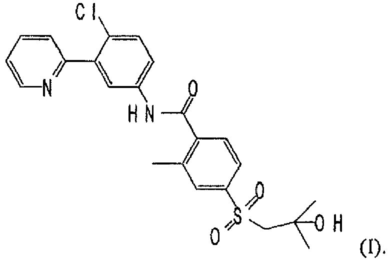

Throughout the specification and the claims the compound having the formula (I) is identical to the compound referred to in claim 1 and its depending claims 2 to 9, as well as referred to in the abstract.

In another aspect of the invention, there is provided a compound of formula (I) for use in a method of treating cancer in a mammal, comprising administering to said mammal an effective amount of a compound of formula (I).

In another aspect of the invention, there is provided a compound of formula (I) for use in a method of treating cancer in a mammal, wherein said cancer is associated with aberrant hedgehog signaling.

In another aspect of the invention, there is provided a compound of formula (I) for use in a method of treating cancer in a mammal, wherein said cancer is basal cell carcinoma, medullablastoma, pancreatic adenocarcinoma, small-cell lung carcinoma, breast carcinoma, rhabdomyosarcoma, oesophageal cancer, stomach cancer or biliary tract cancer.

In another aspect of the invention, there is provided a compound of formula (I) for use in a method of treating cancer in a mammal, wherein said cancer is neuroectodermal, meningioma, hemangioma, glioblastoma, squamous lung carcinoma, non-small cell lung carcinoma, chondrosarcoma, renal carcinoma, thyroid carcinoma or a solid colon, lung, pancreatic, ovarian, breast or glioma tumor.

In another aspect of the invention, there is provided a compound of formula (I) for use in a method of inhibiting angiogenesis in a mammal, comprising administering to said mammal an effective amount of a compound of formula (I).

In another aspect of the invention, there is provided a compound of formula (I) for use in a method of inhibiting hedgehog pathway signaling in a cell, comprising contacting said cell with an effective amount of a compound of formula (I).

In another aspect of the invention, there is provided a compound of formula (I) for use in a method of treating macular degeneration, wet age-related macular degeneration, inflammatory/immune diseases, Crohn's disease, inflammatory bowel disease, Sjogren's syndrome, asthma, organ transplant rejection, systemic lupus erythmatoses, rheumatoid arthritis, psoriatic arthritis, psoriasis or multiple sclerosis or achieving a depilatory effect, comprising administering an effective amount of a compound of formula (I).

In another aspect of the invention, there are provided pharmaceutical compositions comprising the compound of formula (I) and a pharmaceutically acceptable carrier.

In another aspect of the invention, there are provided processes for preparing the compound according to formula (I) of the invention.

### DETAILED DESCRIPTION OF THE PREFERRED EMBODIMENTS

The compound of formula (I) of the invention may contain one or more asymmetric carbon atoms. Accordingly, the compounds may exist as diastereomers, enantiomers or mixtures thereof. The syntheses of the compounds may employ racemates, diastereomers or enantiomers as starting materials or as intermediates. Diastereomeric compounds may be separated by chromatographic or crystallization methods. Similarly, enantiomeric mixtures may be separated using the same techniques or others known in the art. Each of the asymmetric carbon atoms may be in the R or S configuration and both of these configurations are within the scope of the invention.

Also described are prodrugs of the compound described above. Suitable prodrugs include known amino-protecting and carboxy-protecting groups which are released, for example hydrolyzed, to yield the parent compound under physiologic conditions. A particular class of prodrugs are compounds in which a nitrogen atom in an amino, amidino, aminoalkyleneamino, iminoalkyleneamino or guanidino group is substituted with a hydroxy (OH) group, an alkylcarbonyl (-CO-R) group, an alkoxycarbonyl (-CO-OR), an acyloxyalkyl-alkoxycarbonyl (-CO-O-R-O-CO-R) group where R is a monovalent or divalent group and as defined above or a group having the formula -C(O)-O-CP1P2-haloalkyl, where P1 and P2 are the same or different and are H, lower alkyl, lower alkoxy, cyano, halo lower alkyl or aryl. Prodrug compounds may be prepared by reacting the compounds of the invention described above with an activated acyl compound to bond a nitrogen atom in the compound of the invention to the carbonyl of the activated acyl compound. Suitable activated carbonyl compounds contain a good leaving group bonded to the carbonyl carbon and include acyl halides, acyl amines, acyl pyridinium salts, acyl alkoxides, in particular acyl phenoxides such as p-nitrophenoxy acyl, dinitrophenoxy acyl, fluorophenoxy acyl, and difluorophenoxy acyl. The reactions are generally exothermic and are carried out in inert solvents at reduced temperatures such as -78 to about 50 °C. The reactions are usually also carried out in the presence of an inorganic base such as potassium carbonate or sodium bicarbonate, or an organic base such as an amine, including pyridine, TEA, etc. One manner of preparing prodrugs is described in USSN 08/843,369 filed April 15, 1997 (corresponding to PCT publication WO9846576).

### SYNTHESIS

The compound of the invention is prepared using standard organic synthetic techniques from commercially available starting materials and reagents. It will be appreciated that synthetic procedures employed in the preparation of the compound of the invention will depend on the particular substituents present in a compound and that various protection and deprotection procedures may be required as is standard in organic synthesis. The compound of the invention inhibits the hedgehog signaling and is useful to be applied in methods of treatment of cancers associated with aberrant hedgehog signaling, for example when Patched fails to, or inadequately, represses Smoothened (Ptc loss-of-function phenotype) and/or when Smoothened is active regardless of Patched repression (Smo gain-of-function phenotype). Examples of such cancer types include basal cell carcinoma, neuroectodermal tumors such as medullablastoma, meningioma, hemangioma, glioblastoma, pancreatic adenocarcinoma, squamous lung carcinoma, small-cell lung carcinoma, non-small cell lung carcinoma, chondrosarcoma, breast carcinoma, rhabdomyosarcoma, oesophageal cancer, stomach cancer, biliary tract cancer, renal carcinoma, thyroid carcinoma. The Compound of the invention may be administered prior to, concomitantly with, or following administration of other anticancer treatments such as radiation therapy or chemotherapy. Suitable cytostatic chemotherapy compounds include, but are not limited to (i) antimetabolites, such as cytarabine, fludarabine, 5-fluoro-2'-deoxyuiridine, gemcitabine, hydroxyurea or methotrexate; (ii) DNA-fragmenting agents, such as bleomycin, (iii) DNA-crosslinking agents, such as chlorambucil, cisplatin, cyclophosphamide or nitrogen mustard; (iv) intercalating agents such as adriamycin (doxorubicin) or mitoxantrone; (v) protein synthesis inhibitors, such as L-asparaginase, cycloheximide, puromycin or diphteria toxin; (Vi) topoisomerase I poisons, such as camptothecin or topotecan; (vii) topoisomerase II poisons, such as etoposide (VP-16) or teniposide; (viii) microtubule-directed agents, such as colcemid, colchicine, paclitaxel, vinblastine or vincristine; (ix) kinase inhibitors such as flavopiridol, staurosporin, STI571 (CPG 57148B) or UCN-01 (7-hydroxystaurosporine); (x) miscellaneous investigational agents such as thioplatin, PS-341, phenylbutyrate, ET-18- OCH₃, or farnesyl transferase inhibitors (L-739749, L-744832); polyphenols such as quercetin, resveratrol, piceatannol, epigallocatechine gallate, theaflavins, flavanols, procyanidins, betulinic acid and derivatives thereof; (xi) hormones such as glucocorticoids or fenretinide; (xii) hormone antagonists, such as tamoxifen, finasteride or LHRH antagonists. In a particular embodiment, the compound of the present invention is coadministered with a cytostatic compound selected from the group consisting of cisplatin, doxorubicin, taxol, taxotere and mitomycin C.

In another aspect the compound according to formula (I) can be used pursuant to the present invention in a method of treating cancer in a mammal, in which said compound is able to sensitize for or induce apoptosis by binding to death receptors ("death receptor agonists"). Such agonists of death receptors include death receptor ligands such as tumor necrosis factor a (TNF-α), tumor necrosis factor ß (TNF-ß, lymphotoxin-α), LT-ß (lymphotoxin-ß), TRAIL (Apo2L, DR4 ligand), CD95 (Fas, APO-1) ligand, TRAMP (DR3, Apo-3) ligand, DR6 ligand as well as fragments and derivatives of any of said ligands. In a particular embodiment, the death receptor ligand is TNF-α. In another particular embodiment the death receptor ligand is Apo2L/TRAIL. Furthermore, death receptors agonists comprise agonistic antibodies to death receptors such as anti-CD95 antibody, anti-TRAIL-R1 (DR4) antibody, anti-TRAIL-R2 (DR5) antibody, anti-TRAIL-R3 antibody, anti-TRAIL-R4 antibody, anti-DR6 antibody, anti-TNF-R1 antibody and anti-TRAMP (DR3) antibody as well as fragments and derivatives of any of said antibodies.

For the purpose of sensitizing cells for apoptosis, the compound of the present invention can be also used in a method of treating cancer in a mammal in combination with radiation therapy. The phrase "radiation therapy" refers to the use of electromagnetic or particulate radiation in the treatment of neoplasia. Radiation therapy is based on the principle that high-dose radiation delivered to a target area will result in the death of reproducing cells in both tumor and normal tissues. The radiation dosage regimen is generally defined in terms of radiation absorbed dose (rad), time and fractionation, and must be carefully defined by the oncologist. The amount of radiation a patient receives will depend on various considerations including the location of the tumor in relation to other organs of the body, and the extent to which the tumor has spread. Examples of radiotherapeutic agents are provided in, but not limited to, radiation therapy and is known in the art (Hellman, Principles of Radiation Therapy, Cancer, in Principles I and Practice of Oncology, 24875 (Devita et al., 4th ed., vol 1, 1993). Recent advances in radiation therapy include three-dimensional conformal external beam radiation, intensity modulated radiation therapy (IMRT), stereotactic radiosurgery and brachytherapy (interstitial radiation therapy), the latter placing the source of radiation directly into the tumor as implanted "seeds". These newer treatment modalities deliver greater doses of radiation to the tumor, which accounts for their increased effectiveness when compared to standard external beam radiation therapy.

Ionizing radiation with beta-emitting radionuclides is considered the most useful for radiotherapeutic applications because of the moderate linear energy transfer (LET) of the ionizing particle (electron) and its intermediate range (typically several millimeters in tissue). Gamma rays deliver dosage at lower levels over much greater distances. Alpha particles represent the other extreme, they deliver very high LET dosage, but have an extremely limited range and must, therefore, be in intimate contact with the cells of the tissue to be treated. In addition, alpha emitters are generally heavy metals, which limit the possible chemistry and presents undue hazards from leakage of radionuclide from the area to be treated. Depending on the tumor to be treated all kinds of emitters are conceivable within the scope of the present invention. Furthermore, the present invention encompasses types of non-ionizing radiation like e.g. ultraviolet (UV) radiation, high energy visible light, microwave radiation (hyperthermia therapy), infrared (IR) radiation and lasers. In a particular embodiment of the present invention UV radiation is applied in a method of treating cancer in a mammal in combination with administration of a compound having the formula (I).

The compound of the invention inhibits angiogenesis and is therefore useful in a method of treatment of diseases or conditions mediated by angiogenesis such as tumors, in particular solid tumors such as solid colon, lung, pancreatic, ovarian, breast and glioma. Furthermore, the compound of the invention is useful in a method of treating macular degeneration e.g. wet age-related macular degeneration. The Compound of the invention is also useful in a method of treating inflammatory/immune diseases such as Crohn's, inflammatory bowel disease, Sjogren's syndrome, asthma, organ transplant rejection, systemic lupus erythmatoses, rheumatoid arthritis, psoriatic arthritis, psoriasis and multiple sclerosis. The compound of the invention is also useful as a depilatory.

The invention also includes pharmaceutical compositions or medicaments containing the compound of the invention and a therapeutically inert carrier, diluent or excipient, as well as methods of using the compound of the invention to prepare such compositions and medicaments. Typically, the compound of the invention as used in the methods for treatment pursuant to the invention are formulated by mixing at ambient temperature at the appropriate pH, and at the desired degree of purity, with physiologically acceptable carriers, i.e., carriers that are non-toxic to recipients at the dosages and concentrations employed into a galenical administration form. The pH of the formulation depends mainly on the particular use and the concentration of compound, but may range from about 3 to about 8. A particular formulation is an acetate buffer at pH 5. The compound for use herein may be in a sterile formulation. The compound may be stored as a solid composition, although lyophilized formulations or aqueous solutions are acceptable.

The composition of the invention will be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to decrease hedgehog pathway signaling or else is the minimum amount necessary to cause reduction in size, volume or mass of a tumor that is responsive to hedgehog signaling, or a reduction in the increase in size, volume or mass of such a tumor relative to the increase in the absence of administering the compound of the invention. Alternatively "effective amount" of the compound means the amount necessary to reduce the number of malignant cells or the rate in increase of the number of malignant cells. Alternatively, "effective amount" is the amount of the compound of the invention required to increase survival of patients afflicted with an anti-hedgehog pathway sensitive tumor. Such amount may be below the amount that is toxic to normal cells, or the mammal as a whole. With respect to non-malignant indications, "effective amount" means the amount of compound of the invention required to decrease severity of the particular indication or symptoms thereof.

Generally, the initial pharmaceutically effective amount of the compound of the invention administered parenterally per dose will be in the range of about 0.01 to about 100 mg/kg, for example about 0.1 to about 20 mg/kg of patient body weight per day, for example about 0.3 to about 15 mg/kg/day. Oral unit dosage forms, such as tablets and capsules, may contain from about 25 to about 1000 mg of the compound of the invention.

The compound of the invention may be administered by any suitable means, including oral, topical, transdermal, parenteral, subcutaneous, rectal, intraperitoneal, intrapulmonary, and intranasal, and, if desired for local treatment, intralesional administration. Parenteral infusions include intramuscular, intravenous, intraarterial, intraperitoneal, or subcutaneous administration. An example of a suitable oral dosage form is a tablet containing about 25mg, 50mg, 100mg, 250mg, or 500mg of the compound of the invention compounded with about 90-30 mg anhydrous lactose, about 5-40 mg sodium croscarmellose, about 5-30mg polyvinylpyrrolidone (PVP) K30, and about 1-10 mg magnesium stearate. The powdered ingredients are first mixed together and then mixed with a solution of the PVP. The resulting composition can be dried, granulated, mixed with the magnesium stearate and compressed to tablet form using conventional equipment. An aerosol formulation can be prepared by dissolving the compound, for example 5-400 mg, of the invention in a suitable buffer solution, e.g. a phosphate buffer, adding a tonicifier, e.g. a salt such sodium chloride, if desired. The solution is typically filtered, e.g. using a 0.2 micron filter, to remove impurities and contaminants. Topical formulations include ointments, creams, lotions, powders, solutions, pessaries, sprays, aerosols and capsules. Ointments and creams may be formulated with an aqueous or oily base with the addition of suitable thickening and/or gelling agents and/or solvents. Such bases may include water and/or an oil such a liquid paraffin or a vegetable oil such as arachis oil or castor oil or a solvent such as a polyethylene glycol. Thickening agents which may be used include soft paraffin, aluminum stearate, cetostearyl alcohol, polyethylene glycols, microcrystalline wax and beeswax. Lotions may be formulated with an aqueous or oily base and may contain one or more emulsifying agents, stabilizing agents, dispersing agents, suspending agents or thickening agents. Powders for external application may be formed with the aid of any suitable powder base e.g. talc, lactose or starch. Drops may be formulated with an aqueous or non-aqueous base also comprising one or more dispersing agents, solubilizing agents or suspending agents.

### EXAMPLES

The invention will be more fully understood by reference to the following examples. This should not, however, be construed as limiting the scope of the invention defined by the appended claims. Abbreviations used herein are as follows:
BuOH: butanol;
DIPEA: diisopropylethylamine;
DMA: NN-dimethylacetamide;
DMAP: 4- dimethylaminopyridine;
DME: 1,2-dimethoxyethane;
DMF: dimethylformamide;
EDC: 1-ethyl-3-(3-dimethylaminopropyl)carbodiimide;
HATU: O-(7-Azobenzotriazol-1-yl)-1,1,3,3-tetramethyluronium hexafluorophosphate;
HPLC: high pressure liquid chromatography
MPLC: medium pressure liquid chromatography
NBS: N-Bromosuccinimide;
TEA: Triethylamine;
TASF: tris(dimethylamino)sulfonium difluorotrimethylsilicate;
THF: tetrahydrofuran;
EtOH: Ethanol;
MeOH: Methanol;
µL: microlitre

All reagents were obtained commercially unless otherwise noted. Reactions were performed using oven-dried glassware under an atmosphere of nitrogen. Air and moisture sensitive liquids and solutions were transferred via syringe or stainless steel cannula. Organic solutions were concentrated under reduced pressure (ca. 15 mm Hg) by rotary evaporation. Unless otherwise noted all solvents used were obtained commercially. Chromatographic purification of products was accomplished by use of an Isco CombiFlash Companion and media. Reaction times are given for illustration only. The course of reactions was followed by thin-layer chromatography (TLC) and liquid chromatography-mass spectrometry (LC-MS). Thin-layer chromatography (TLC) was performed on EM Science silica gel 60 F₂₅₄ plates (250 µm). Visualization of the developed chromatogram was accomplished by fluorescence quenching. LC-MS were acquired with a Shimadzu 10AD LC on a Phenomenex column (50 x 4.6 mm, 5 µm) operating at 3 mL/min. A Shimadzu SPD-10A detector monitoring at 214 and 254 nm was used. Single quadrupole mass spectrometry was performed on an Applied Biosystems mass spectrometer. Nuclear magnetic resonance (NMR) spectra were acquired on a Varian Inova spectrometer operating at 400 MHz for ¹H and are referenced internally to tetramethylsilane (TMS) in parts per million (ppm). Data for ¹H NMR are recorded as follows: chemical shift (δ, ppm), multiplicity (s, singlet; bs, broad singlet; d, doublet; t, triplet; q, quartet; quint, quintet; sext, sextet; hept, heptet; m, multiplet; bm, broad multiplet), and integration. The structure and purity of all final products were assessed by at least one of the following techniques: LC-MS, NMR, TLC.

### Example 1 General Procedures

The Compound of below example 2 was prepared according to the following general procedure.

### A: Amide bond coupling with HATU

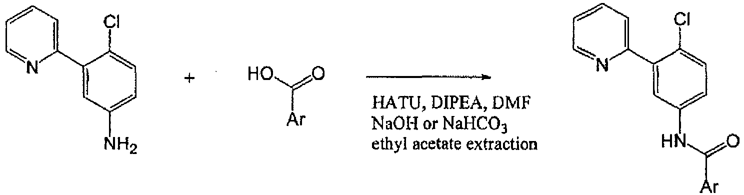

Aniline (1.0 eq) was added to a mixture of carboxylic acid (1.1 eq), HATU (1.1 eq) and DIPEA (2 eq) in DMF (0.1 - 0.2 M). After stirring overnight, the reaction mixture was diluted with 0.1 N sodium hydroxide or saturated NaHCO₃, extracted into ethyl acetate and the combined organic layers were washed with brine. The organic layer was dried (MgSO₄), concentrated and the crude mixture was purified by reverse phase HPLC.

### Example 2 (a) N-(4-chloro-3-(pyridin-2-yl)phenyl)-4-(ethylsulfonyl)benzamide

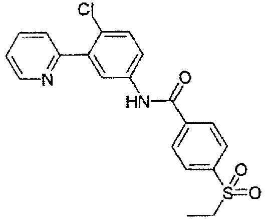

General procedure A was performed using 4-(ethylthio)benzoic acid and 4-chloro-3-(pyridin-2-yl)aniline to produce *N*-(4-chloro-3-(pyridin-2-yl)phenyl)-4-(ethylthio)benzamide.

A solution of *N*-(4-chloro-3-(pyridin-2-yl)phenyl)-4-(ethylthio)benzamide (40 mg, 0.11 mmol) in MeOH (3 mL), cooled to 0° C was treated with oxone (133 mg, 0.22 mmol), and the ice bath was removed. After 1h of stirring, the reaction mixture was concentrated, and the residue was dissolved in ethyl acetate. The organic solution was washed with water, dried (MgSO₄) and concentrated. The crude reaction mixture was purified by reverse phase HPLC to yeild *N-*(4-chloro-3-(pyridin-2-yl)phenyl)-4-(ethylsulfonyl)benzamide. MS (Q1) 401.0 (M)⁺.

### (b) N-(4-chloro-3-(pyridin-2-yl)phenyl)-4-(2-hydroxy-2-methylpropylsulfonyl)-2-methylbenzamide

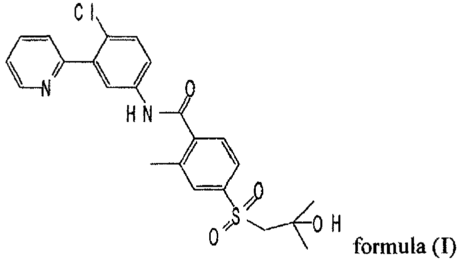

2-Chloro-5-nitroiodobenzene: The reactor used was purged with nitrogen and kept under nitrogen throughout the synthesis. Reactor was charged with USP purified water (400.0 L), agitated and charged with 2-chloro-5-nitroaniline (50.0 kg) and then the contents were cooled to 0 - 5 °C. To the stirring reactor was charged concentrated sulfuric acid (40.0 L), maintaining the temperature at ≤10 °C (addition time ∼3-4 hr) and the contents were stirred at 0 - 5 °C for at least 15 minutes. In a separate vessel a solution of sodium nitrite (25.0 kg) and USP purified water (100.0L) was prepared. The sodium nitrite solution was slowly charged to the stirred reactor maintaining the temperature at ≤ 5 °C (exotherm and caused gas evolution, addition time -2 hours) and then the contents were stirred at ≤ 5 °C for at least 1 hour. In a separate vessel a solution of potassium iodide (60.0 kg) and USP purified water (240.0 L) was prepared and slowly charged to the stirred reactor maintaining the temperature at ≤5 °C (exotherm, caused gas evolution and foaming, addition time ∼7 hr). Cooling was turned off gradually allowing reaction to reach room temperature (∼20 °C) and then the contents were stirred for at least 18 hours at 15-25 °C, and then sampled reaction mass by HPLC analysis (dissolved sample in acetonitrile), when ≤ 5% of 2-chloro-5-nitroaniline remained then continued to next step, however when the level of starting material was ≥ 5% then sampled every hour until the reaction was complete. In a separate vessel a solution of sodium thiosulfate (30.0 kg) and USP purified water (600.0 L) was prepared and slowly charged ∼1/2 of the sodium thiosulfate solution to the stirred reactor, maintaining the temperature at 20 - 30 °C and then stirred reactor contents at 20 - 30 °C for at least 20 minutes. Cyclohexane (300.0 L) was charged to the reactor and the contents were heated to 55 - 60 °C and stirred for at least 20 minutes at 55 - 60 °C. Agitation was stopped to allow the layers to settle for at least 10 minutes and then were separated (setting aside organic layer) and returned the aqueous layer back into reactor. Cyclohexane (200.0 L) was charged to the reactor and stirred at 55 - 60 °C for at least 20 minutes and then agitation was stopped to allow the layers to settle for at least 10 minutes and then separating the layers (held aqueous layer for yield check) and combined both the organic layers from previous steps back into the reactor. The remaining ∼1/2 of the sodium thiosulfate solution was charged to the stirred reactor, maintaining temperature at 55 - 60 °C and stirred for at least 20 minutes at 55 - 60 °C. Agitation was stopped to allow the layers to settle for at least 10 minutes and the the aqueous layer was drained from the reactor. USP purified water (300.0 L) was then charged to the reactor and stirred for at least 20 minutes at 55 - 60 °C and then agitation was stopped to allow the layers to settle for at least 10 minutes and the aqueous layer was drained to waste. The reactor contents were heated at ∼45 °C. and removed ∼65% of the solvent by vacuum distillation. Reactor contents were then cooled to 0 - 5 °C and allowed to stir for at least 5 hours and then the solids were filtered and the product was washed with cold cyclohexane (100.0 L). The product was collected and dried in a hot air drier at 45 +/- 5 °C until LOD was ≤ 1.0%. The process yielded 50.0 kg (61 % yield) of 2-chloro-5-nitroiodobenzene as a yellow solid.

Crude 2-(2-pyridyl)-4-nitrochlorobenzene: Reactor was purged with nitrogen and kept under nitrogen throughout the synthesis. Toluene (375.0 L) was charged to the reactor and agitation was begun and zinc chloride (19.56 kg) was charged to the reactor. Using atmospheric distillation reactor contents was stripped to ∼50% of the original volume and then cooled to ≤ 30 °C. THF (100.0 L) was slowly charged to the reactor (addition was exothermic).

Preparation of Grignard reagent in reactor 2: Reactor was purged with nitrogen and kept under nitrogen throughout the synthesis. Agitation was begun and THF (50.0 L) was charged to reactor. Isopropyl magnesium chloride (89.0 kg, adjusted after titration) was drum rolled to mix and then was slowly added, maintaining the temperature at ≤ 30 (exothermic, addition time 30-40 min). 2-bromopyridine (22.3 kg) was slowly charged to the reactor maintaining the temperature at ≤ 30 oC (exothermic, addition time 50-60 min). The reactor contents were then heated to 50 +/- 5 °C and maintained for at least 1 hour. The Grignard solution (from Reactor 2) was slowly charged to the reactor (from earlier step) maintaining the temperature at ≤ 55 °C (exothermic addition caused foaming, addition time ∼20 min). The reactor was then stirred at 50 +/- 5 °C at least 1 hour while maintaining the temperature. Dichlorobistriphenylphosphine palladium (2.0 kg) was charged to the reactor and stirred for ∼15 minutes. Triphenylphosphine (2.75 kg) was charged to the reactor and stirred for ∼15 minutes. 2-chloro-5-nitroiodobenzene (25.0 kg) was slowly charged to the stirred reactor (15 minute addition time). The reactor contents were heated to 60 +/- 5 °C and stirred for at least 14 hours at 60 +/- 5 °C, then sampled the reaction mass for HPLC analysis. When amount of starting material was ≥ 4% continued heating and sampled again every hour until the level of starting material fell below 4%. The reaction mixture was cooled to ∼55 °C. and then the reactor contents were heated to reflux under vacuum and 75-90 L of solvent was removed. Toluene (120.0 L) was charged to the reactor while stirring. In a separate tank, the ammonium chloride (25.0 kg) was dissolved in USP purified water (250.0 L) and the solution was slowly charged into the reactor and stir for at least 30 minutes. The mixture was filtered through a Nutsche filter (prepared with Celite (6.25 kg) and USP purified water (12.5 L)) and the filter cake was washed with toluene (75.0 L) and the filtrate was added and washed into a clean reactor. The layers were allowed to settle for at least 10 minutes and then separated (organic layer contained product) and returned the aqueous layer to the reactor. Toluene (75.0 L) was charged to the reactor and stirred for at least 15 minutes and then the layers were allowed to settle for at least 10 minutes before separating (organic layer contained product). The organic layers from previous steps were charged to a clean reactor. USP purified water (125.0 L) was charged to the reactor and stir for at least 15 minutes and then the layers were allowed to settle for at least 10 minutes before draining the aqueous layer and holding for yield check.

In a separate tank, a 3N hydrochloric acid solution was prepared by mixing concentrated hydrochloric acid (127.5 L) and USP purified water (272.5 L). Approximately 1/3 of the 3N hydrochloric acid (133.3 L) was charged to the reactor and stir for at least 30 minutes. The layers were allowed to settle for at least 15 minutes and then the aqueous layer was drained and transferred to a separate vessel (product was in aqueous layer). Approximately 1/3 of the 3N hydrochloric acid (133.3 L) was charged to the reactor and stirred for at least 30 minutes. The layers were allowed to settle for at least 15 minutes and then the aqueous layer was drained and transferred to a separate vessel (product was in aqueous layer). Aproximately 1/3 of the 3N hydrochloric acid (133.3 L) was charged to the reactor and stirred for at least 30 minutes. The layers were allowed to settle for at least 15 minutes and then the aqueous layer was drained and transferred to a separate vessel (product was in aqueous layer). The aqueous layers from previous steps were transferred into a clean reactor to which was charged activated carbon (1.0 kg) and then heated to 50 +/- °C and stirred for at least 30 minutes. The mixture was filtered through a Nutsche filter (prepared with Celite (5.0 kg) and USP purified water (12.5 L)) and the filter cake was washed with 3N hydrochloric acid (40.0 L) and the filtrate was added and washed into a clean reactor. The combined aqueous solutions were polish filtered through a 1 micron filter into a clean reactor and cooled to ≤ 10 °C. Ammonium hydroxide (115.0 L) was slowly charged to the reactor, adjusting the pH to between 8.5 and 9.0 (addition time 4.25 hours). The reaction temperature was adjusted to 25-30 °C and the mixture was allowed to stir for 30 minutes. Reaction mixture was then centrifuged and the product washed with USP purified water (300.0 L) and dried in a hot air dryer at 50 - 60 °C. Process yielded 15.0 kg (72%) of crude 2-(2-pyridyl)-4-nitrochlorobenzene.

Purification of 2-(2-pyridyl)-4-nitrochlorobenzene - The reactor was purged with nitrogen and kept under nitrogen throughout the synthesis. Dichloromethane (400.0 L) was charged to the reactor and stirring was begun. Crude 2-(2-pyridyl)-4-nitrochlorobenzene (40.0 kg) was charged to the reactor and stirred at 20 - 30 °C for at least 30 minutes and checked to see if all solids were dissolved. Silica gel (20.0 kg) was charged to the reactor and stirred for at least 2 hours. The mixture was filtered through a Nutsche filter (prepared with Celite (14.8 kg) and dichloromethane (14.8 L)) and the filter cake was washed with dichloromethane (80.0 L) and the filtrate was added and washed into a clean reactor. The reactor contents were heated to reflux under vacuum and 80-90% of the solvent was removed and the cooled to 20 - 30 °C and then n-hexane (240.0 L) was charged to the reactor which was stirred for at least 2 hours at 20 - 30 °C. The reaction mixture was filted and washed with n-hexane (80.0 L) and the product dried in a hot air dryer at 50 - 55 °C. Process yields 34.5 kg (86% recovery) of 2-(2-pyridyl)-4-nitrochlorobenzene as a beige solid.

4-chloro-3-(pyridin-2-yl)aniline: 2-(2-pyridyl)-4-nitrochlorobenzene was charged to a suitably sized reactor under nitrogen. Platinum on carbon (5%, ∼50% wet) (0.10 wt) was added with stirring, followed by tetrahydrofuran (9.68 wt). The reactor was pressurized with nitrogen to 40psi, then the pressure was released. This process was repeated two additional times. The reactor was then pressurized with hydrogen to 50psi while maintaining the internal temperature at 20-26°C. After the hydrogen uptake subsided (1-2 hours), the pressure was held at 50psi and the reactor was heated to 50°C for 2-3 hours. The reaction was checked by HPLC and once complete, cooled to 30°C. Next, the reactor was pressurized with nitrogen to 40psi, then the pressure was released. This process was repeated two additional times. To a separate tank, Celite (0.1 wt) and tetrahydrofuran (0.9 wt) were added. This slurry was then transferred to the reactor and stirred for a minimum of 30 minutes. The reaction mixture was filtered through a filter press and 0.2 micron filter, the cake was washed with tetrahydrofuran (2.2 wt) and all the organics were combined. Thiol silica gel (0.05 wt) was charged to the reactor and stirred for at least 30 minutes. This mixture was then filtered through a filter press into an adjacent, nitrogen purged, reactor. The filter cake was washed with tetrahyrdofuran (2.2 wt) and the wash was added back to the reactor. With stirring, heptanes (6.8 wt) were added to the reactor and the contents were heated to reflux under vacuum. Approximately two thirds of the solvent was removed by vacuum distillation. The reactor was cooled to 20-26°C and stirred for 2-3 hours. The reactor contents were centrifuged and washed with heptanes (1.0 wt) and dried in a vacuum oven at 20-25°C until a constant weight of 4-chloro-3-(pyridin-2-yl)aniline was obtained (typical yield ∼80%).

*N*-(4-chloro-3-(pyridin-2-yl)phenyl)-4-(2-hydroxy-2-methylpropylsulfonyl)-2-methylbenzamide-Tetrahydrofuran (10.24 wt) was charged to a suitably sized reactor under nitrogen. While stirring, 4-(2-hydroxy-2-methylpropylsulfonyl)-2-methylbenzoic acid (1.265 wt) and 2-chloro-4,6-dimethoxy-1,3,5-triazine (0.815 wt) were added and stirred until dissolved. 4-methylmorpholine (0.564 wt) was slowly charged to the reactor while maintaining the internal temperature at ≤ 30°C. The mixture was allowed to stir at room temperature for at least 30 minutes then sampled by TLC. Once all of the 4-(2-hydroxy-2-methylpropylsulfonyl)-2-methylbenzoic acid was consumed, 4-chloro-3-(pyridin-2-yl)aniline (1.0 wt) was added. The reactor was heated to 50°C and stirred for at least 6 hours, at which time the reaction was sampled by HPLC. Once the reaction was complete by HPLC, a sodium bicarbonate solution (sodium bicarbonate (0.506 wt) and USP purified water (24.8 wt), stirred until all solids were dissolved) was added to the reaction. The reaction mixture was heated to reflux (∼70°C) and solvent (5.7 wt.) was distilled from the reactor. The reactor was cooled to ≤ 30°C and stirred for at least 20 hours. The reactor contents were centrifuged, washed with USP purified water (3.47 wt) and dried in a vacuum oven at 45 °C until a constant weight of crude *N*-(4-chloro-3-(pyridin-2-yl)phenyl)-4-(2-hydroxy-2-methylpropylsulfonyl)-2-methylbenzamide was obtained (typical yield ∼90%).

Methyl isobutyl ketone (20.0 wt) was charged to a suitably sized reactor under nitrogen. While stirring, crude *N*-(4-chloro-3-(pyridin-2-yl)phenyl)-4-(2-hydroxy-2-methylpropylsulfonyl)-2-methylbenzamide (1.0 wt) was added and the reactor was heated to 60 °C and stirred for at least one hour. The solution was polish filtered through a filter press into an adjacent, nitrogen purged, reactor and the cake was washed with methyl isobutyl ketone (2.56 wt.). The filtered solution was then heated to reflux (∼115 °C) and distilled to remove ∼2/3 of the solvent (∼14.5 wt). The reactor was cooled to 100 °C and stirred for at least 15 minutes. The reactor was then cooled to 80 °C and the tip speed of the agitator was set to 2.0 m/s. A seed slurry was prepared by mixing *N*-(4-chloro-3-(pyridin-2-yl)phenyl)-4-(2-hydroxy-2-methylpropylsulfonyl)-2-methylbenzamide Form A (0.001 wt) and methyl isobutyl ketone (0.008 wt). This seed slurry was added to the reactor at 80 °C and stirred for at least 2.5 hours. The bath temperature was set to 70 °C and the contents were stirred until the internal temperature reached 70 °C. The bath temperature was set to 50 °C and the contents wee stirred until the internal temperature reached 50 °C. The bath temperature was set to 25 °C and the contents were stirred until the internal temperature reached 15-30 °C. Once this temperature was obtained, the mixture wa stirred for at least 12 hours. In a separate tank, a solution was prepared by charging methyl isobutyl ketone (3.0 wt) and heptanes (2.6 wt). The reactor contents were centrifuged, washed with the methyl isobutyl ketone/heptanes mixture (all) and dried in a vacuum oven at 60 °C until a constant weight of purified *N*-(4-chloro-3-(pyridin-2-yl)phenyl)-4-(2-hydroxy-2-methylpropylsulfonyl)-2-methylbenzamide was obtained. The solids were milled using a Fitzmill grinder utilizing an 18 mesh screen, hammers forward on low speed. (typical yield ∼80%).

### Example 3 Hedgehog signalling inhibition assays

Mouse Reporter Cell lines - 10T1/2-GliLuc [S12] cells (derived from cell line C3H10T1/2 ATCC #CCL-226) ; Mouse Embryonic Fibroblasts); Growth Medium: Dulbecco's modified Eagles' Medium (DMEM) supplemented with 10% Fetal Bovine Serum (FBS), 10 units/mL penicillin, 100 ug/mL streptomycin, 2mM glutamine, and 10mM HEPES.

Human Reporter Cell lines - HEPM-GliLuc [MZ24] - cells (derived from HEPM, Human Embryonic Palatal Mesenchyme ATCC #CRL-1486); Growth Medium: Minimum Essential Medium (MEM; with Earle's salts) supplemented with 10-20% Fetal Bovine Serium (FBS), 10 units/mL penicillin, 100ug/mL streptomycin, 2mM glutamine, and 10mM HEPES pH 7.2.

Sonic hedgehog - recombinant human SHh N-terminal octylated conjugate.

Microtiter Plates (MTPs) - For the Luciferase assay cells are plated in 96-well MTPs (White, Flat-bottom, Clear-View).

Luciferase-Assay Medium - DMEM supplemented with 0.5% FBS, 10 units/mL penicillin, 100ug/mL streptomycin, 2mM glutamine, and 10mM HEPES pH 7.2.

PBS/Ca/Mg Mix - Phosphate Buffered Saline (PBS) supplemented with 0.5mM CaCl₂ and 1mM MgCl₂.

### Assay Procedure

S12 and MZ24 cells genetically modified to contain a luciferase reporter gene driven by the hedgehog-reseponsive Gli promoter were maintained on tissue culture dishes in Growth Medium at 37°C and 5% CO₂. Cell cultures were passaged at sub-confluency at every 3-4 days. (1:20 to 1:40 for s12; 1:3 to 1:10 for MZ24). Cells were harvested and diluted in Growth Medium such that they could be plated in a microtitre plate at 10,000-20,000 cells (s12), or 20,000-30,000 cells (MZ24), per 100ul, per well. Cells were further incubated for ∼24-48 hours at 37°C and 5% CO₂.

After ∼24-48 hour incubation the Growth Medium in the microtitre plates was replaced by Luciferase-Assay Medium (100 ul per well), with and without Sonic hedgehog-octyl conjugate, at 0.1-0.3 ug/ml (S12) or 0.5-1.0 ug/ml (MZ24), and test compounds. Cells were then further incubated for and additional 24 hrs.

Microtitre plates were then subjected to the luciferase reporter gene assay kit (LucLite™), with modifications to the manufacturer's procedure wherein medium was removed and the substrate was reconstituted with 1:1 PBS/Ca/Mg : lysis buffer instead of straight lysis buffer. In brief, the PBS/Ca/Mg was mixed 1:1 with lysis buffer and 10mL were added to each substrate vial (of the 1000-assay kit). Then the assay media from the microtitre plate was discarded, and 100ul of this substrate mix was added to each well. Plates were incubated at room termperature for 20-30 minutes and then the Relative Light Units (RLUS) representing the relative expression level of the luciferase reporter gene were determined with a Topcount reader (Packard) or an Analyst reader (Molecular Devices). Compounds of the invention tested in the assays demonstrated reduced Gli expression in the reporter cell lines indicating hedgehog pathway signalling inhibition.

## Claims

1. A compound of the formula

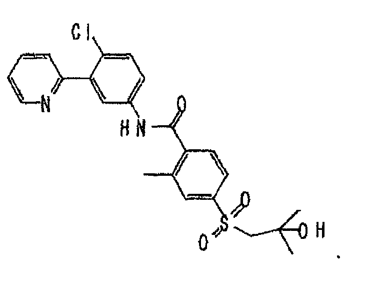

2. The compound of claim 1 for use in a method of treating cancer in a mammal, comprising administering to said mammal an effective amount of a compound of claim 1.

3. The compound of claim 1 for use in a method of treating cancer in a mammal, wherein said cancer is associated with aberrant hedgehog signaling.

4. The compound of claim 1 for use in a method of treating cancer in a mammal, wherein said cancer is basal cell carcinoma, medullablastoma, pancreatic adenocarcinoma, small-cell lung carcinoma, breast carcinoma, rhabdomyosarcoma, oesophageal cancer, stomach cancer or biliary tract cancer.

5. The compound of claim 1 for use in a method of treating cancer in a mammal, wherein said cancer is neuroectodermal, meningioma, hemangioma, glioblastoma, squamous lung carcinoma, non-small cell lung carcinoma, chondrosarcoma, renal carcinoma, thyroid carcinoma or a solid colon, lung, pancreatic, ovarian, breast or glioma tumor.

6. The compound of claim 1 for use in a method of inhibiting angiogenesis in a mammal, comprising administering to said mammal an effective amount of a compound of claim 1.

7. The compound of claim 1 for use in a method of inhibiting hedgehog pathway signalling in a cell, comprising contacting said cell with an effective amount of a compound of claim 1.

8. The compound of claim 1 for use in a method of treating macular degeneration, wet age-related macular degeneration, inflammatory/immune diseases, Crohn's disease, inflammatory bowel disease, Sjogren's syndrome, asthma, organ transplant rejection, systemic lupus erythmatoses, rheumatoid arthritis, psoriatic arthritis, psoriasis or multiple sclerosis or achieving a depilatory effect, comprising administering an effective amount of a compound of claim 1.

9. A pharmaceutical composition comprising a compound of claim 1 and a phamaceutically acceptable carrier.

## Patentansprüche

1. Verbindung der Formel

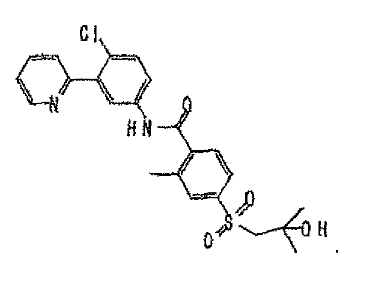

2. Verbindung nach Anspruch 1 für die Anwendung in einem Verfahren zur Krebsbehandlung bei einem Säugetier, das die Verabreichung einer effektiven Menge der Verbindung nach Anspruch 1 an das Säugetier umfasst.

3. Verbindung nach Anspruch 1 für die Anwendung in einem Verfahren zur Krebsbehandlung bei einem Säugetier, wobei der Krebs mit einer aberranten Hedgehog-Signalübertragung assoziiert ist.

4. Verbindung nach Anspruch 1 für die Anwendung in einem Verfahren zur Krebsbehandlung bei einem Säugetier, wobei der Krebs ein Basalzellkarzinom, Medulloblastom, ein pankreatisches Adenokarzinom, kleinzelliges Lungenkarzinom, Brustkarzinom, Rhabdomyosarkom, Speiseröhrenkrebs, Magenkrebs oder Gallengangskrebs ist.

5. Verbindung nach Anspruch 1 für die Anwendung in einem Verfahren zur Krebsbehandlung bei einem Säugetier, wobei der Krebs ein neuroektodermaler Tumor, ein Meningiom, Hämangiom, Glioblastom, Plattenepithelkarzinom der Lunge, ein nicht-kleinzelliges Lungenkarzinom, Chondrosarkom, Nierenkarzinom, Schilddrüsenkarzinom oder ein fester Dickdarm-, Lunge-, Bauchspeicheldrüsen-, Eierstock-, Brusttumor oder ein Gliom ist.

6. Verbindung nach Anspruch 1 für die Anwendung in einem Verfahren zur Angiogenese-Hemmung bei einem Säugetier, das die Verabreichung einer effektiven Menge der Verbindung nach Anspruch 1 an das Säugetier umfasst.

7. Verbindung nach Anspruch 1 für die Anwendung in einem Verfahren zur Hemmung des Hedgehog-Signalweges in einer Zelle, das den Kontakt der Zelle mit einer effektiven Menge der Verbindung nach Anspruch 1 umfasst.

8. Verbindung nach Anspruch 1 für die Anwendung in einem Verfahren zur Behandlung von Makuladegeneration, feuchter altersbedingter Makuladegeneration, Entzündungs-/Immunerkrankungen, Morbus Crohn, entzündlicher Darmerkrankung, Sjögren Syndrom, Asthma, Organtransplantatabstoßung, systemischem Lupus erythematodes, rheumatoider Arthritis, Psoriasis-Arthritis, Psoriasis oder multipler Sklerose oder zum Erreichen eines Enthaarungseffekts, das die Verabreichung einer effektiven Menge einer Verbindung nach Anspruch 1 umfasst.

9. Pharmazeutische Zusammensetzung, die eine Verbindung nach Anspruch 1 und einen pharmazeutisch verträglichen Träger umfasst.

## Revendications

1. Composé de formule

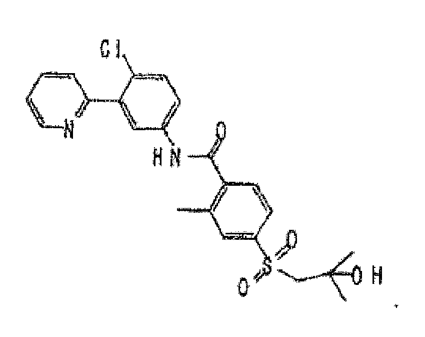

2. Composé selon la revendication 1 pour une utilisation dans une méthode de traitement du cancer chez un mammifère, comprenant l'administration au dit mammifère d'une quantité efficace d'un composé selon la revendication 1.

3. Composé selon la revendication 1 pour une utilisation dans une méthode de traitement du cancer chez un mammifère, dans lequel ledit cancer est associé à une signalisation hedgehog aberrante.

4. Composé selon la revendication 1 pour une utilisation dans une méthode de traitement du cancer chez un mammifère, dans lequel ledit cancer est un carcinome à cellules basales, un médulloblastome, un adénocarcinome pancréatique, un carcinome du poumon à petites cellules, un carcinome du sein, un rhabdomyosarcome, un cancer de l'oesophage, un cancer de l'estomac ou un cancer des canaux biliaires.

5. Composé selon la revendication 1 pour une utilisation dans une méthode de traitement du cancer chez un mammifère, dans lequel ledit cancer est neuroectodermique, un méningiome, un hémangiome, un glioblastome, un carcinome du poumon à cellules squameuses, un carcinome du poumon non à petites cellules, un chondrosarcome, un carcinome rénal, un carcinome de la thyroïde ou une tumeur solide du côlon, du poumon, pancréatique, de l'ovaire, du sein ou de gliome.

6. Composé selon la revendication 1 pour une utilisation dans une méthode d'inhibition de l'angiogenèse chez un mammifère, comprenant l'administration au dit mammifère d'une quantité efficace d'un composé selon la revendication 1.

7. Composé selon la revendication 1 pour une utilisation dans une méthode d'inhibition de la voie de signalisation hedgehog dans une cellule, comprenant la mise en contact de ladite cellule avec une quantité efficace d'un composé selon la revendication 1.

8. Composé selon la revendication 1 pour une utilisation dans une méthode de traitement de la dégénérescence maculaire, de la dégénérescence maculaire humide liée à l'âge, de maladies inflammatoires/immunitaires, de la maladie de Crohn, de maladies inflammatoires de l'intestin, du syndrome de Sjögren, de l'asthme, d'un rejet de transplantation d'organe, du lupus érythémateux systémique, de la polyarthrite rhumatoïde, de l'arthrite psoriasique, du psoriasis ou de la sclérose en plaques ou d'obtention d'un effet dépilatoire, comprenant l'administration d'une quantité efficace d'un composé selon la revendication 1.

9. Composition pharmaceutique comprenant un composé selon la revendication 1 et un support pharmaceutiquement acceptable.
